# EUROPEAN PATENT SPECIFICATION

(11) **EP 1 312 369 B1**
(45) Date of publication and mention of the grant of the patent: **11.11.2009**
(21) Application number: 01945633.4
(22) Date of filing: 27.06.2001
(51) Int. Cl.: A61K 31/7016, A61K 33/14, A61P 9/00

(54) **CARDIAC ARREST FLUID**
FLÜSSIGKEIT FÜR HERZSTILLSTAND
LIQUIDE POUR L'ARRET CARDIAQUE

(30) Priority: 26.07.2000 JP 2000225330
(43) Date of publication of application: 21.05.2003
(73) Proprietor: FUSO PHARMACEUTICAL INDUSTRIES LTD., Osaka-shi, Osaka 541-0045 (JP)
(72) Inventor: KOMEDA, Masashi, Kamigyo-ku, Kyoto-shi, Kyoto 602-8024 (JP); MIWA, Senri, Nishinomiya-shi, Hyogo 662-0037 (JP); WADA, Hiromi, Otsu-shi, Shiga 520-0865 (JP)
(74) Representative: Hubert, Philippe
(86) International application number: PCT/JP2001/005508
(87) International publication number: WO 2002/007735

(56) References cited:
- EP-A- 0 580 444
- EP-A1- 0 580 444
- WO-A1-00/64254
- WO-A1-98/41213
- JP-A- 3 294 201
- JP-A- 9 151 134
- BAUDOT A ET AL: "Effects of saccharides on the glass-forming tendency and stability of solutions of 2,3-butanediol, 1,2-propanediol, or 1,3-butanediol in water, phosphate-buffered saline, Euro-Collins solution, or Saint Thomas cardioplegic solution" CRYOBIOLOGY, vol. 33, no. 3, 1996, pages 363-375, XP002365448 ISSN: 0011-2240
- EMMOTO T ET AL: "Effect of trehalose on long-term supercool preservation of cultured rat cardiac myocytes" CRYOBIOLOGY, vol. 33, no. 6, 1996, page 643, XP002365449 & THIRTY-THIRD ANNUAL MEETING OF THE SOCIETY FOR CRYOBIOLOGY; INDIANAPOLIS, INDIANA, USA; AUGUST 17-21, 1996 ISSN: 0011-2240
- SHIROISHI MARK S: "Myocardial protection: The rebirth of potassium-based cardioplegia" TEXAS HEART INSTITUTE JOURNAL, vol. 26, no. 1, 1999, pages 71-86, XP002365450 ISSN: 0730-2347

## Description

### Technical Field

The present invention relates to a cardioplegic solution.

### Background Art

In heart surgery, the heart needs to be subjected to artificial cardiac standstill by perfusing the heart with a cardioplegic solution. In addition, during cardiac standstill, the cardiac muscle is in a state of ischemia resulting in a lack of oxygen, which is likely to reduce heart functions. Thus, a cardioplegic solution must be provided with a function of protecting the heart muscle in addition to the action of stopping the heart. Conventionally known cardioplegic solutions include the St. Thomas solution. The St. Thomas solution contains a variety of metal ions such as Na⁺, K⁺, Mg⁺⁺, and Ca⁺⁺, and the concentration of each ion is close to that of the extracellular fluid.

However, conventional cardioplegic solutions do not have a sufficient action of protecting the cardiac muscle.

Hence, the object of the present invention is to provide a cardioplegic solution useful for cardiac standstill and cardiac muscle protection.

### Disclosure of the Invention

The present inventors have conducted intensive studies and completed a solution for use in cardioplegia of the present invention, which contains trehalose and metal ions. A solution for use in cardioplegia of the present invention is provided with an excellent action of protecting the cardiac muscle in comparison with the conventional ones.

As trehalose, any of α,α-trehalose, α,β-trehalose, and β, β-trehalose may be used. Desirably, α,α-trehalose present in nature is used. The content of trehalose is from 1.5 to 3.5% (w/v).

Metal ions that are made contained in a cardioplegic solution of the present invention include Na⁺, K⁺, Mg⁺⁺, and Ca⁺⁺, because they induce a sudden heart stop or prevent the inflow of extracellular metal ions into the inside of the cell while maintaining the balance with the extracellular fluid. Regarding contents of these, Na⁺ is from 100 to 140 mmol/L, K⁺ is from 10 to 20 mmol/L, Mg⁺⁺ is from 5 to 40 mmol/L, and Ca⁺⁺ is from 0.5 to 3 mmol/L. Also, addition of electrolytes such as NaCl, KC1, MgCl₂-6H₂O, and CaCl₂-2H₂O is appropriate in order to render these metal ions to be contained therein.

The pH of a cardioplegic solution of the present invention is desirably in the range of 7.4 to 7.8, which is close to the range of the extracellular fluid. To adjust the pH to this range, addition of a suitable amount of, for example, NaHC03 is appropriate.

### Best Mode for Carrying out the Invention

### - Examples -

NaCl, KCl, MgCl₂-6H₂O, CaCl₂-2H₂O, and α, α-trehalose are dissolved in water and then to this was added water to totally 100 mL. Thereafter, to the resulting solution was added 1 mL of an 8.4% (w/v) NaHCO₃ solution to prepare Example Solutions 1 to 3 indicated in Table 1. In addition, a St. Thomas solution was prepared in a similar manner except using no α,α-trehalose. Additionally, the contents in Table 1 express the values measured prior to the addition of the NaHCO₃ solution.

**[Table 1]**

| | | Thomas solution | Example Solution 1 | Example e Solution 2 | Example Solution 3 |
|---|---|---|---|---|---|
| Attributes | Color and clarity Osmotic pressure (m0sm/kg) | Colorless and clear 294 | Colorless and clear 358 | Colorless and clear 390 | Colorless and clear 427 |
| | Transmittivity (%) | 100.0 | 100.0 | 100.0 | 100.0 |
| pH | | 7.80 | 7.43 | 7.44 | 7.78 |
| Contents | Na⁺ (mmol/l) | 112.6 | 110.4 | 110.0 | 112.7 |
| | K⁺ (mmol/l) | 16.3 | 15.8 | 15.7 | 16.4 |
| | Ca⁺⁺(mmol/l) | 1.12 | 1.18 | 1.16 | 1.12 |
| | Mg" (mmol/l) | 16.3 | 15.5 | 15.7 | 16.3 |
| | C1 (mmol/l) | 161.8 | 161.0 | 161.4 | 162.1 |
| | Trehalose (w/v%) | - | 2.03 | 3.01 | 4.16 |
| Insoluble foreign matter | Not observed | Not observed | Not observed | Not observed | Not observed |

### - Test Example 1 -

SD rats (male) with weights of 300 to 400 g are divided into two groups (Group A and Group B), each having 9 rats, and then are subjected to the cardiac standstill test below. In addition, as the cardioplegic solution, the St. Thomas solution was used for Group A and Example Solution 2 was used for Group B.

First, each rat was anesthetized with ether and subjected to median thoractomy. Then, the heart was removed and immediately immersed in an ice cooled Krebs-Henseleit Buffer (NaCl 0.6895 g, KCl 0.0343 g, MgSO₄ 0.145 g, CaCl₂-2H₂O 0.368 g, KH₂PO₄ 0.164 g, NaHCO₃ 2.10 g, Glucose 0.188 g/100 mL). Immediately after the weight measurement, the heart was placed in the Langendorff apparatus and then was subjected to preliminary perfusion using Krebs-Henseleit Buffer (saturated with a mixture gas of O₂ (95%) and CO₂ (5%)) as the perfusion solution. Thereafter, a latex balloon was inserted at the left atrium into the left ventricle by way of the mitral valve and further was connected to a pressure monitor via a transducer. After 20-minute preliminary perfusion, the balloon was made expanded in the left ventricle for pressure measurement, thereby evaluating the pressure-volume relationship.

Next, the heart was made stopped by perfusion of 20 mL/kg of a cardioplegic solution cooled to 4°C. The cardioplegic solution was added by 10 mg/kg every 30 minutes. After the cardiac standstill was kept for 90 minutes, the Krebs-Henseleit Buffer (saturated with a mixture gas of O₂ (95%) and CO₂ (5%)) was re-perfused as the perfusion solution. Then, at a lapse of 5 minutes after the initiation of the re-perfusion, a pressure-volume relationship was evaluated in the same manner as the above, with the ratio of the value after to the value before, the cardiac standstill, calculated. In addition, the weight of the cardiac muscle was measured to evaluate the weight increase ratio relative to the value prior to the cardiac standstill and further the cardiac muscle was observed pathologically.

As a result, the ratios of the end-diastolic pressure-volume relationship (EDPVR) values to the values prior to the cardiac standstill were 1.59±0.90 for Group A and 3.45±1.16 for Group B, indicating that there is no significant difference between the two groups (P = 0.13). However, regarding the end-systole pressure-volume relationship (ESPVR), the ratios were 0.71±0.07 for Group A and 0.98±0.05 for Group B, indicating that the value of Group B is significantly better (P < 0.05). This shows that Example Solution 2 is superior in the action of maintaining cardiac muscle systole ability to the St. Thomas solution.

Furthermore, the weight increase ratios of the cardiac muscle relative to the value prior to the cardiac standstill were 1.46±0.71 for Group A and 1.14±0.37 for Group B, showing that Group B significantly indicates a smaller value (P < 0.001). This shows that Group B has a fewer occurrences of edema. In addition, pathological observation also shows that Group B tends to have a fewer occurrences of edema. This proves that Example Solution 2 is better in action of suppressing edema than the St. Thomas solution.

Consequently, Example Solution 2 is shown to be superior in action of cardiac muscle protection to the St. Thomas solution. Table 1 shows that Example Solution 2 has almost the same composition as that of the St. Thomas solution except containing trehalose. Therefore, the test results show that trehalose has an excellent action of protecting the cardiac muscle.

### - Test Example 2 -

SD rats (male) with weights of 300 to 400 g are divided into three groups (Group a, Group b and Group c), each having 7 rats. Then, in a manner similar to Test Example 1 above, the heart of each rat was made stopped and subjected to re-perfusion. The ratios of the pressure-volume relationship relative to the value prior to cardiac standstill were evaluated at lapses of 5 minutes and 15 minutes after the initiation of the re-perfusion. In addition, regarding cardioplegic solutions, Example Solution 1 was used for Group a, Example Solution 2 for Group b, and Example Solution 3 for Group c.

As a result, the ratios of the end-systole pressure-volume relationship were found to have no significant differences between the three groups at lapses of both 5 minutes and 15 minutes. The ratios of the end-diastolic pressure-volume relationship, at a lapse of 15 minutes, were 1.67 ± 1.07 for Group a, 1.88 ± 0.65 for Group b, and 1.74 ± 1.23 for Group c, which show no significant differences as well. However, the ratios, at a lapse of 5 minutes, were 3.25 ± 1.43 for Group a, 3.45 ± 1.16 for Group b, and 5.74 ± 2.88 for Group c, with the value of Group c being inferior to those of the other two groups. In other words, Group c has a slow recovery of the cardiac muscle diastole ability as compared with the other two groups. This shows that Example Solutions 1 and 2 are superior in action of maintaining the cardiac muscle diastole ability to Example Solution 3. In addition, Table 1 indicates that the osmotic pressure values of Example Solutions 1 and 2 are as good as 358 and 390 mOsm/kg, respectively, while the value of Example Solution 3 is as high as 427 mOsm/kg.

Consequently, Example Solutions 1 and 2 are more appropriate as the cardioplegic solution than Example Solution 3. Table 1 indicates that Example Solutions 1, 2, and 3 contain, respectively, about 2, about 3, and about 4.1% (w/v) of trehalose, and they have almost the same composition excluding the concentrations of trehalose. Thus, the test results suggest that a suitable concentration of trehalose in a cardioplegic solution is from about 1.5 to about 3.5% (w/v).

### Industrial Applicability

A cardioplegic solution of the present invention is provided with excellent actions of maintaining heart function and of suppressing edema, and thus is useful for cardiac standstill and cardiac muscle protection.

## Claims

1. A solution containing a trehalose in an amount of 1.5 to 3.5% (W/V). Na⁺ in an amount of 100 to 140 mmol/L, K⁺ in an amount of 10 to 20 mmol/L, Mg⁺⁺ in amount of 5 to 40 mmol/L and Ca⁺⁺ in an amount of 0.5 to 3 mmol/L for use in cardioplegia.

2. The solution according to claim 1, wherein the trehalose is α,α-trehalose.

3. The solution according to claim 1 or claim 2, wherein the metal ion of Na⁺, K⁺, Mg⁺⁺ and Ca+⁺ is derived from an electrolyte.

4. The solution according to any one of claims 1, 2 or 3, wherein the solution has a pH of 7.4 to 7.8.

## Patentansprüche

1. Lösung enthaltend eine Trehalose in einer Menge von 1,5 bis 3,5 % (Gewicht/Volumen), Na⁺ in einer Menge von 100 bis 140 mmol/L, K⁺ in einer Menge von 10 bis 20 mmol/L, Mg⁺⁺ in einer Menge von 5 bis 40 mmol/L und Ca⁺⁺ in einer Menge von 0,5 bis 3 mmol/L zur Benutzung bei der Kardioplegie.

2. Lösung nach Anspruch 1, wobei die Trehalose α,α-Trehalase ist.

3. Lösung nach einem der Ansprüche 1 oder 2, wobei das Metall-lon von Na⁺, K⁺, Mg⁺⁺ und Ca⁺⁺ aus einem Elektrolyt stammt.

4. Lösung nach einem der Ansprüche 1, 2 oder 3, wobei die Lösung einen pH-Wert von 7,4 bis 7,8 besitzt.

## Revendications

1. Solution contenant un tréhalose en une quantité de 1,5 à 3,5 % (M/V), Na⁺ en une quantité de 100 à 140 mmol/L, K⁺ en une quantité de 10 à 20 mmol/L, Mg⁺⁺ en une quantité de 5 à 40 mmol/L et Ca⁺⁺ en une quantité de 0,5 à 3 mmol/L destinée à être utilisée en cardioplégie.

2. Solution selon la revendication 1 où le tréhalose est l'α,α-tréhalose.

3. Solution selon la revendication 1 ou la revendication 2 où l'ion métallique Na⁺, K⁺, Mg⁺⁺ et Ca⁺⁺ est dérivé d'un électrolyte.

4. Solution selon l'une quelconque des revendications 1, 2 et 3 où la solution a un pH de 7,4 à 7,8.
